**Europäisches Patentamt**

**European Patent Office**

**Office européen des brevets**

(11) Veröffentlichungsnummer: **0 483 401 B1**

(12) **EUROPÄISCHE PATENTSCHRIFT**

(45) Veröffentlichungstag der Patentschrift: **05.10.94**

(51) Int. Cl.[5]: **A61M 16/00**, G05D 7/06

(21) Anmeldenummer: **90120843.9**

(22) Anmeldetag: **30.10.90**

(54) **Anordnung bzw. Ventilator zur Regelung der Durchflussmenge eines fliessenden Mediums, insbesondere eines Gases.**

(43) Veröffentlichungstag der Anmeldung:
**06.05.92 Patentblatt 92/19**

(45) Bekanntmachung des Hinweises auf die Patenterteilung:
**05.10.94 Patentblatt 94/40**

(84) Benannte Vertragsstaaten:
**CH DE ES FR GB IT LI NL SE**

(56) Entgegenhaltungen:
**CH-A- 430 837**
**GB-A- 2 026 326**
**US-A- 3 972 327**

(73) Patentinhaber: **Siemens Elema AB**
**Röntgenvägen 2**
**S-171 95 Solna 1 (SE)**

(84) Benannte Vertragsstaaten:
**SE**

(73) Patentinhaber: **SIEMENS AKTIENGESELL-SCHAFT**
**Wittelsbacherplatz 2**
**D-80333 München (DE)**

(84) Benannte Vertragsstaaten:
**CH DE ES FR GB IT LI NL**

(72) Erfinder: **Skog, Göran, Dipl.-Ing.**
**Hildebandsvägen 32**
**S-161 53 Broma (SE)**
Erfinder: **Lindén, Dan, Dipl.-Ing.**
**Dianavägen 35**
**S-115 43 Stockholm (SE)**
Erfinder: **Cewers, Göran, Dipl.-Ing.**
**Möllevongsvägen 89**
**S-222 40 Lund (SE)**
Erfinder: **Inderup, Mats, Dipl.-Ing.**
**Hässleholmsgatan 7**
**S-214 43 Malmö (SE)**
Erfinder: **Dahlström, Bo, Dipl.-Ing.**
**Alvdalsvägen 48A**
**S-162 42 Vällingby (SE)**
Erfinder: **Rydgren, Göran, Dipl. Ing.**
**Strandängsvägen 4,**
**S-230 44 Bunkeflostrand (SE)**

**Beschreibung**

Die Erfindung betrifft eine Anordnung gemäss dem Oberbegriff des Anspruchs 1. Eine derartige Anordnung ist beispielsweise aus der US-Patentschrift 3,741,208 bekannt. Der darin beschriebene Ventilator steuert den Gasfluss zu bzw. von einem Patienten mit Hilfe eines analogen elektronischen Regelkreises mit negativem Feedback. Von einem Sollwertgeber wird ein einstellbarer Referenzwert für den zeitlichen Verlauf des gewünschten Gasflusses erzeugt. Ein Gasflussmesser bestimmt den Istwert für den aktuellen Gasfluss und wandelt ihn in ein entsprechendes elektrisches Signal um, das nach einer Linearisierung einem Eingang eines Differenzbildners zugeführt wird , auf dessen anderen Eingang der vom Sollwertgeber gelieferte Referenzwert geschaltet ist. Das Ausgangssignal des Differenzbildners bildet den proportionalen Teil der Regelgrösse, die zur Ansteuerung eines Schrittmotors dient, mit dem über einen Exzenter eine schlauchförmige Gasleitung im Querschnitt derart verändert wird, dass die Differenz zwischen dem aktuellen und dem gewünschten Gasfluss gegen Null strebt.Dem proportionalen Teil der Regelgrösse ist in bekannter Weise ein integraler Teil zugefügt.

Mit diesem Ventilator gelang es erstmals, verschiedene zeitabhängige Beatmungsmuster reproduzierbar zu realisieren.

In dem Prospekt über den PB 7200 Microprocessor Ventilator, Puritan-Bennett Corp., Form No. AA-213 (5/83) ist eine entsprechende digitale Regelung beschrieben, bei der der mit einem Heissfilmmanometer analog gemessene aktuelle Gasfluss digitalisiert und zwischengespeichert wird, um dann in einem Mikroprozessor mit einem für den gewünschten Gasfluss gespeicherten Tabellenwert als Sollwert zur Berechnung einer Regelgrösse verwendet zu werden. Die Regelgrösse setzt sich dabei aus einem proportionalen, einem differentialen und einem integralen Anteil zusammen, zu denen eine Konstante addiert ist, mit deren Hilfe das Festsitzen der Verschlussmittel an der Ventilöffnung verhindert wird. Als Ventil dient ein Solenoidventil, wie es beispielsweise aus der US-Patentschrift Nr. 4,463,332 bekannt ist. Dabei ist als Verschlussmittel ein längliches Teil aus magnetischem Material vorgesehen, dass mit Hilfe einer federnden Membran, die eine lineare Federkraft auf das Teil ausübt, gegen die Ventilöffnung gedrückt wird. Beim Anlegen eines Stromes an die Spule des Solenoidventiles wird eine Kraft als Funktion des magnetischen Flusses entgegen der Federkraft erzeugt, die das Teil von der Ventilöffnung abhebt und diese entsprechend der erreichten Gleichgewichtslage freigibt. Der Aufbau des Ventiles ist dabei so gewählt, dass die Bewegung des Teiles linear proportional dem angelegten elektrischen Strom ist.

In beiden bekannten Ventilatoren ist jeweils ein einziger Regelkreis mit negativem Feedback zur Steuerung der Lage der Verschlussmittel vorgesehen, um den Gasfluss zu regeln.

Weiterhin ist aus der CH-A-0 430 837 eine Regeleinrichtung mit einem elektromagnetischen Stellorgan z.B. für die Einstellung der Durchflußmenge durch ein Ventil bekannt. In einem einzigen Regelkreis wird dabei aus einem Istwert für die Lage des Stellorgans und einem Sollwert ein Fehlersignal ermittelt, daß über einen Verstärker den Strom durch die Spule eines Elektromagneten derart schaltet, daß sich der Istwert dem Sollwert nähert. Um das unerwünschte Schwingen derartiger Systeme um eine Mittelwertlage zu dämpfen, ist hier vorgeschlagen, dem Fehlersignal ein Stabilisierungssignal zu überlagern, das beispielsweise vom Strom des Elektromagneten abhängig sein kann.

Darüber hinaus ist aus einem Artikel in **Aerospace Medicine,Vol.36,No.11,Nov.1965** mit dem Titel **Some Aspects of the Dynamic Behaviour of Aircrew Breathing Equipment** ein Atmungssimulator bekannt, bei dem der gewünschte Gasfluss nicht über ein Ventil, sondern über eine Kolbenbewegung gesteuert wird und bei dem der Versuch einer Regelung angedeutet ist. Als Istgrössen werden dabei der Gasfluss, die Lage des Kolbens sowie die Geschwindigkeit des Motors, mit dem der Kolben angetrieben wird, bestimmt. Alle drei Istwerte werden jedoch mit einem einzigen Sollwert, dem von einem "Wave-form generator" erzeugten Referenzsignal für den zeitlichen Verlauf des gewünschten Gasflusses in einem einzigen Regelkreis verglichen, d.h. die erhalten elektrischen Signale für die verschiedenen Istwerte werden gemeinsam von dem Referenzsignal subtrahiert. Eine einwandfreie Regelung ist dabei unter normalen Bedingungen nicht möglich, da sich die unterschiedlichen Istwerte gegenseitig stören. So kann der Istwert für den Gasfluss beispielsweise über dem Sollwert liegen und darauf hinwirken, den aktuellen Fluss zu vermindern, der Istwert für die Lage des Kolbens dagegen einen zu niedrigen Gasfluss indizieren und genau in die entgegengesetzte Richtung wirken, d.h. auf eine Erhöhung des Gasflusses hin.

Der vorliegenden Erfindung liegt die Aufgabe zugrunde, eine Anordnung der eingangs genannten Art und insbesondere einen Ventilator so zu verbessern, dass bei hoher Regelgenauigkeit schnell der angestrebte Wert für die Durchflussmenge erreicht wird. Insbesondere werden dabei Zeiten von kleiner als 10 ms angestrebt.

Eine weitere Aufgabe besteht darin, die nötige elektrische Leistung zum Betreiben der Anordnung bzw. des Ventilator so klein wie möglich zu halten.

Weiterhin soll die Anordnung bzw. der Ventilator innerhalb eines grösseren Druckbereiches des dem Ventil zugeführten Mediums einwandfrei arbeiten.

Zusätzlich liegt der Erfindung die Aufgabe zugrunde, dass bei Wegfall einer Regelgrösse das Ventil sicher und schnell schliesst, um eine unkontrollierte Abgabe des Mediums, insbesondere die Abgabe von Atem- oder Anästhesiegas von einem Ventilator zu verhindern.

Diese Aufgaben werden durch die in den Ansprüchen angegebenen Merkmale gelöst, wobei die Unteransprüche darüber hinaus zusätzliche Vorteile der Erfindung angeben.

Im folgenden wird ein Ausführungsbeispiel der Erfindung anhand von sechs Figuren näher beschrieben und erläutert. Dabei zeigen

Fig.1 eine Prinzipskizze eines Magnetventiles,
Fig.2 ein Diagramm über die Abhängigkeit des Gasflusses von der Lage der Verschlussmittel
Fig.3 ein erstes Blockschema eines erfindungsgemässen Ventilators,
Fig.4 ein weiteres Blockschema des Ventilators,
Fig.5 eine Schaltung zur impulsbreitenmodulierten Ansteuerung des Magnetventiles und
Fig.6a-c die zeitlichen Verläufe der Ist- und Sollwerte für den Strom sowie die dazugehörige Steuerspannung.

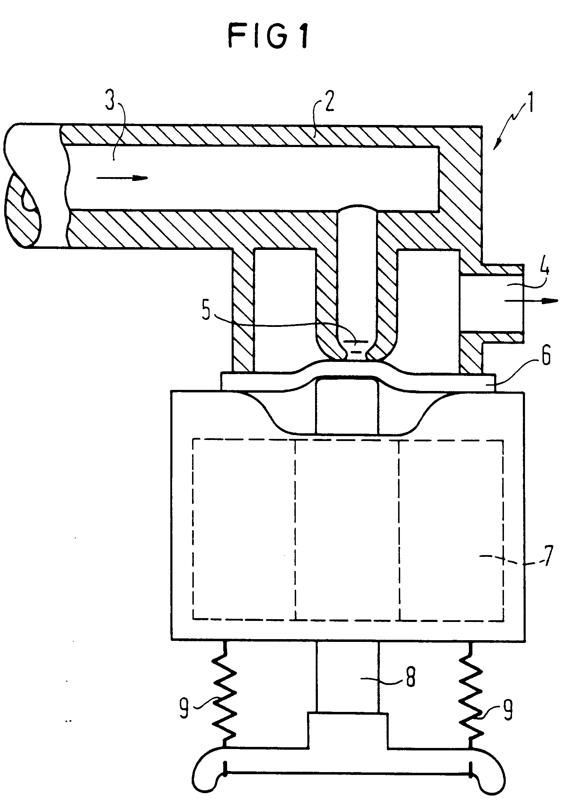

In Fig. 1 ist teilweise geschnitten eine mögliche Ausführungsform eines Magnetventieles **1** als Prinzipskizze dargestellt. Das Magnetventil **1** weist ein Gehäuse **2** auf, in dem sich ein durchgehender Kanal für ein zu regulierendes Gas befindet, der in einen Einlass **3**, einen Auslass **4** und eine dazwischen angeordnete Ventilöffnung **5** unterteilt ist. Gegenüber der Ventilöffnung **5** befindet sich eine Membran **6** aus einem speziellen Gummi oder entsprechendem Material, die zum Verschliessen der Ventilöffnung **5** dient. Unterhalb der Membran **6** befindet sich eine gekapselte Magnetspule **7** in der zentrisch verschiebbar eine Stange **8**,beispielsweise aus rostfreiem Stahl, gelagert ist, die im Bereich der Spule mit magnetischem Material ummantelt ist. Mittels Zugfedern **9** wird die Stange **8**, wenn die Magnetspule **7** nicht erregt ist, mit ausreichender Kraft gegen die Membran **6** gedrückt, so dass die Ventilöffnung **5** sicher verschlossen ist. Im Rahmen der Erfindung kann diese Kraft auch mit Druckfedern erzielt werden. In beiden Fällen wird ein angestrebter Sicherheitsaspekt erzielt, dass nämlich bei einem Stromausfall das Magnetventil **1** schliesst und eine unkontrollierte Atemgasabgabe beispielsweise an einen Patienten verhindert wird.

Wenn nun die Magnetspule **7** durch einen Strom erregt wird, wird die Stange **8** nach unten in die Spule hineingezogen und die Membran **6** hebt entsprechend durch eigene Federkraft und durch den Gasdruck auf der Einlasseite von der Ventilöffnung **5** ab.

Bei überkritischen Druckverhältnissen (grosser Unterschied zwischen dem Druck auf der Einlass- und Auslasseite) an einer Ventilöffnung ist der Gasfluss proportional zur Querschnittsfläche der Öffnung sowie dem Quotienten (Absolutdruck Einlass) / (Absolutdruck Auslass). Wenn durch Anlegen unterschiedlicher Stromstärken der Abstand der Membran **6** von der Ventilöffnung **5** verändert wird, wird auch die effektive Querschnittsfläche dieser Öffnung und damit der Gasfluss verändert. Die von der Magnetspule **7** auf die Stange **8** ausgeübte Kraft ist dabei annähernd proportional dem Strom durch die Spule **7**. Durch Wahl des richtigen Arbeitspunktes können magnetische Hysterese und Unlinearitäten weitgehend vermieden werden.Friktion in den Lagern, in denen die Stange **8** geführt ist, und die verbleibende magnetische Hysterese bewirken jedoch, dass der Strom beim Öffnen des Ventiles **1** in eine bestimmte Position anders ist als der Strom beim Schliessen des Ventiles **1** in die gleiche Position. Bei sehr kleinen Gasflüssen ist die Position nicht mehr linear zum Strom, da die Membran **6** als zusätzliche Feder wirkt.

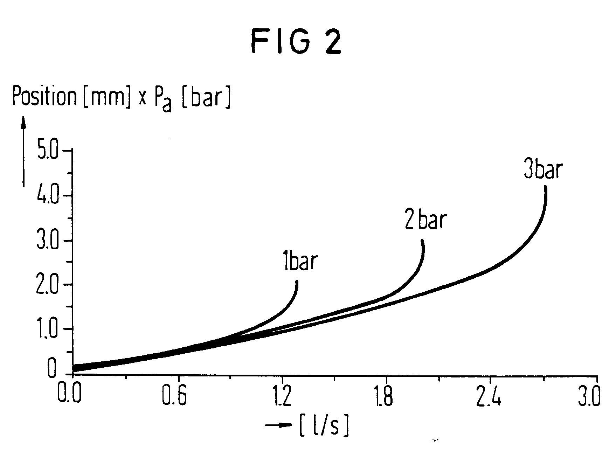

Fig.2 zeigt in einem Diagramm die Abhängigkeit des Gasflusses von der Position der Stange **8** multipliziert mit dem absoluten Gasdruck auf der Einlasseite für drei verschiedene Drücke, 1 bar, 2 bar und 3 bar. Wie diesem Diagramm zu entnehmen ist, ist bei einem gegebenen Druck der Gasfluss über weite Bereiche annähernd proportional der Position.

Ein Ventilator mit nur einem Regelkreis für den Gasfluss, wobei die erzeugte Regelgrösse direkt den an die Magnetspule **7** angelegten Strom steuert, erfordern eine Reihe von Eigenschaften, um überhaupt funktionieren zu können. Die Hysterese muss kompensiert werden, wozu aber die mögliche Verstärkung des Regelkreises nicht ausreicht. Um das Ventil einigermassen schnell zu machen, muss die Verstärkung mit dem Gasfluss kräftig geändert werden. Sie muss für jeden Gasfluss genau eingestellt sein, anderenfalls fängt der Regelkreis an zu schwingen.

Insgesamt ist damit nur eine begrenzte Einstellschnelligkeit erzielbar. Mit einem nur den Gasfluss berücksichtigenden Regelkreis ist das Hauptproblem die schwingfähige Gassäule, die bei zu grosser Regelverstärkung die Tendenz zum Überschwingen hat. Erzielbare Einstellzeiten liegen , wie Versuche ergeben haben, bei etwa 20 ms.

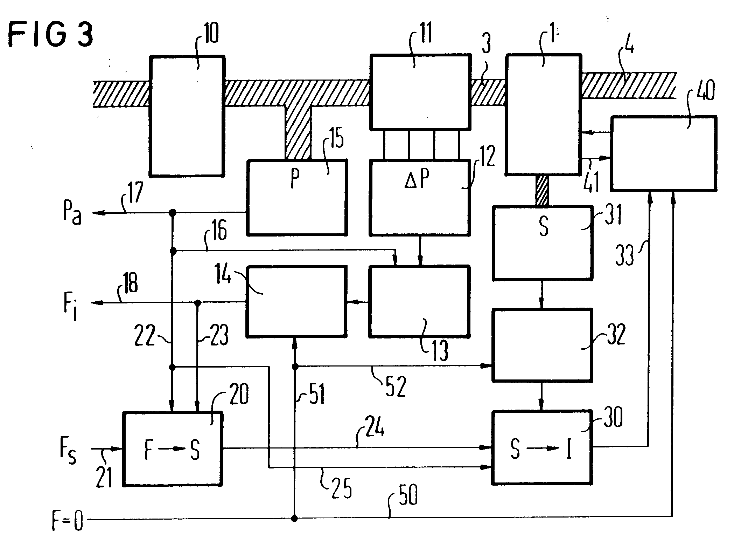

Wegen dieser Erkenntnis wurde für die erfindungsgemässe Anordnung bzw. den Erfindungsgemässen Ventilator eine andere Regelstrategie gewählt, die an Hand eines in Fig.3 dagestellten Blockschemas erläutert werden soll.

In dieser Fig. ist das Magnetventil **1** mit Gaseinlass **3** und Gasauslass **4** schematisch als Block dargestellt. Das Gas, beispielsweise Atemgas, wird dem Gaseinlass **3** von einer nicht dargestellten Gasquelle über ein Filter **10** und eine Vorrichtung zur Bestimmung des Istwertes der Durchflussmenge, im folgenden kurz Gasflussmesser genannt, geführt. Dieser Gasflussmesser kann in bekannter Weise aus einem Röhrchenpaket **11** bestehen, in dem es zu einem definierten Druckabfall p kommt, sowie einem Druckmesser **12**. Dieser Druckabfall wird aus der Differenz zwischen dem Druck auf der Eingangsseite und dem auf der Ausgangsseite des Röhrchenpaketes **11** ermittelt. Der aktuelle Gasfluss, d.h. der Istwert $F_i$ lässt sich dann als das Produkt von $\Delta p \times P_a$ berechnen, wobei $P_a$ der eingangsseitige Gasdruck ist, der mit einem Gasdruckmesser **15** bestimmt wird. Anstelle des Röhrchenpaketes kann vorteilhafterweise auch eine Art Netz verwendet werden, das ausserdem noch mit Fasern versehen sein kann. der Druckabfall erfolgt dadurch über eine sehr kurze Strecke, was die Geschwindigkeit des Gasflussmessers steigert.

In Fig.3 wird das Ausgangssignal des Blockes **12** in einem Block **13**, dem zusätzlich über eine Leitung **16** der Gasdruck $P_a$ zugeführt wird, linearisiert und über einen weitern Block **14**, der einen Nullpunksabgleich in bekannter Weise durchführt, über eine Leitung **18** an eine hier nicht dargestellte Gasflussanzeige geleitet. Der eingangsseitige Gasdruck $P_a$ wird über eine Leitung **17** einer nicht dargestellten Druckanzeige zugeführt.

Weiterhin ist in Fig.3 ein erster Regelkreis **20** für den Fluss dargestellt, dem über eine Leitung **21** von einem nicht dagestellten Referenzwertgeber ein dem Sollwert für den gewünschten Fluss $F_s$ entsprechendes Signal und über Leitungen **22,23** der Gasdruck $P_a$ und der Istwert des Flusses $F_i$ zugeführt werden. Der erste Regelkreis erzeugt mit diesen Eingangswerten ein Regelsignal S, das die Differenz zwischen Soll- und Istwert für den Fluss zu Null machen soll. Dieses Regelsignal S wird über eine Leitung **24** dem zweiten Regelkreis **30** als Sollwert $S_s$ für die Position der Verschlussmittel, in diesem Falle der Stange **8** gemäss Fig.1, zugeführt.Über eine Leitung **25** wird dem Regelkreis **30** der Druck $P_a$ zugeführt. Eine Vorrichtung für die Bestimmung der Lage der Verschlussmittel, kurz Lagesensor **31** genannt, ermittelt die aktuelle Position der Stange **8**. Über eine Block **32**,der dem Nullabgleich dient, wird dieser Wert als Istwert $S_i$ für die Position der Stange **8** dem Regelkreis **30** zugeführt, der aus den eingegebenen Werten eine Regelgrösse I berechnet, die als Sollwert $I_s$ einem weiteren Regelkreis **40** zur Stromregelung über eine Leitung **33** zugeführt wird. Da der eingangsseitige Gasdruck $P_a$ eine Kraft auf die Membran **6** gemäss Fig.1 in Öffnungsrichtung ausübt, soll der Strom durch die Magnetspule **7** und damit der Sollwert $I_s$ in Abhängigkeit von diesem Gasdruck $P_a$ veränderbar sein.

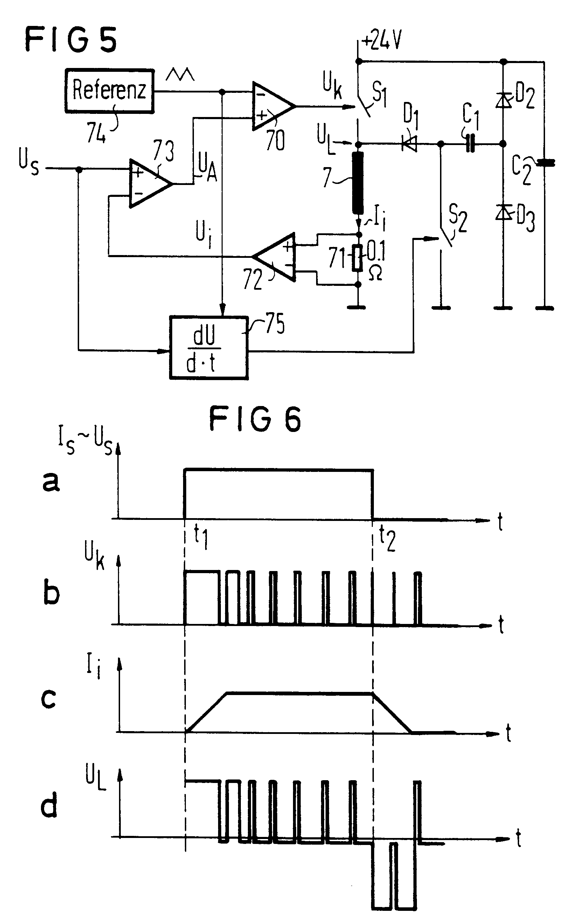

In dieser Blockdarstellung enthält der Block **1** neben dem Magnetventil auch noch eine Messvorrichtung zum Bestimmen des Istwertes für den Strom $I_i$. Dieser Istwert wird dem Regelkreis **40** über eine Leitung **41** zugeführt. Dieser Regelkreis ermittelt aus der Differenz zwischen Ist- und Sollwert eine Regelgrösse $U_A$, die, wie später an Hand der Fig. 5 näher beschrieben wird, zur impulsbreitenmodulierten Stromsteuerung des Magnetventiles **1** verwendet wird.

Zusätzlich ist in Fig.3 noch eine Leitung **50** vorhanden, über die von einer nicht dargestellten Signalquelle ein Signal für den Gasfluss Null auf den Regelkreis **40** gegeben werden kann. Dieses Signal wird über Leitungen **51,52** auch den Blöcken **14,32** für den Nullabgleich zu deren Aktivierung zugeführt.

Dieses Regelsystem, bei dem gewissermassen drei Regelkreise in Reihe geschaltet sind, bietet eine Reihe von Vorteilen. So kann die Verstärkung und die Bandbreite jedes Regelkreises optimal eingestellt werden. Alle Möglichkeiten hoher Verstärkung und grosser Bandbreite können ausgenutzt werden, was zu einer insgesamt sehr viel schnelleren Regelung führt. So kann beispielsweise der Regelkreis **40** für den Strom eine grosse Bandbreite haben, wenn die Induktanz und der Wiederstand der Spule des Magnetventiles **1** niedrig gehalten werden.

Der Regelkreis **30** für die Position der Stange **8** kann eine höhere Verstärkung aufweisen , als das bei einem entsprechenden Regelkreis für den Gasfluss möglich wäre. Wie Fig. 2 ausserdem gezeigt hat, ist die Position linear proportional dem Fluss, was bedeutet, dass die richtige Position, d.h. der Sollwert für den Regelkreis **30** als $F_s/P_a$ berechnet werden kann.Der Regelkreis **20** für den Gasfluss dient dann zur Feinjustierung.

Weitere Vorteile des erfindungsgemässen Regelsystems bestehen darin, dass die Fehlerdiagnostik und der Abgleich erleichtert werden und die Sicherheit des Systems erhöht wird. Beispielsweise kann der Sollwert für die Position der Stange **8** zusammen mit dem eingangsseitigen Gasdruck zur Kontrolle des Gasflussmessers verwendet werden.

Zusammengefasst lässt sich die Funktion des in Fig.3 beispielhaft dargestellten Ventilators folgendermassen beschreiben:
Vier Parameter des Ventiles werden gemessen, nämlich der eingangsseitige Gasdruck $P_a$, der Druckabfall $\Delta p$ über einer definierten Verengung, die Position $S_i$ der Verschlussmittel und der Strom $I_i$ durch die Magnetspule. Ausserdem wird aus $P_a$ und $\Delta p$ der Istwert für den Fluss $F_i$ berechnet.

Das Magnetventil wird von dem Signal für den Sollwert des Gasflusses gesteuert. Der erste Regelkreis **20** für den Gasfluss bildet mit Hilfe von $F_s$, $F_i$ und $P_a$ das Signal $S_s$. Der zweite Regelkreis **30** für die Position der Verschlussmittel bildet mit Hilfe der Signale $S_s$, $S_i$ unf $P_a$ das Signal $I_s$. Der weitere Regelkreis **40** für den Strom durch die Magnetspule **1** bildet aus $I_s$ und $I_i$ ein impulsbreitenmoduliertes Signal, das der Spule zugeführt wird.

Die folgenden Formeln geben beispielhaft an, wie die einzelnen Regelgrössen berechnet werden können:

$$S_s = S_{sN} + S_{sP} + S_{sI}$$

mit

$$S_{SN} = \frac{F_s}{P_a} \times k_1$$

$$S_{sP} = ( F_s - F_i ) \times k_2$$
$$S_{sI} = \int (F_s - F_i) \times k_3$$

und

$$I_s = I_{sP} + I_{sD} + I_{sPa} + I_4$$

mit

$$I_{sP} = ( S_s - S_i ) \times k_4$$

$$I_{sD} = \frac{d(-S_i)}{dt} \times k_5$$

$$I_{sPa} = -P_a \times k_6$$

$$I_4 = k_7$$

und

$$U_A = ( I_s - I_i ) \times k_8$$

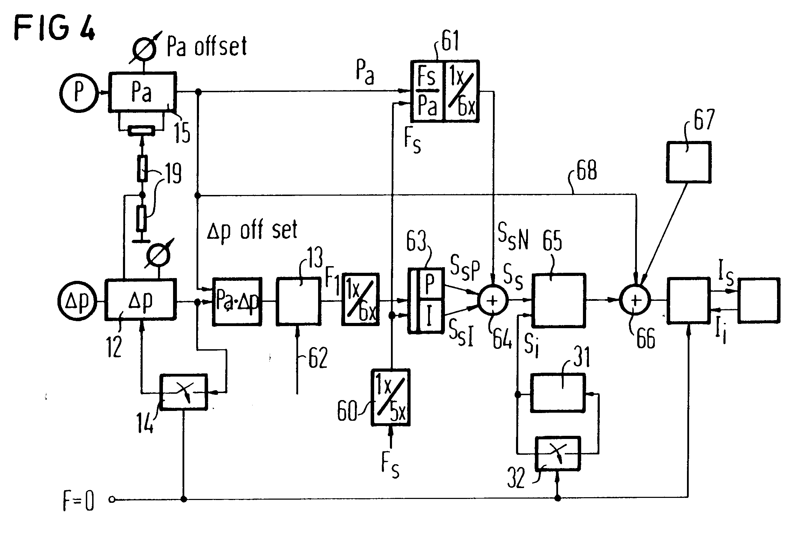

In der Fig.4 ist ein weiteres Blockschema des erfindungsgemässen Ventilators mehr im Detail dargestellt, wobei, soweit möglich, Bezug auf die Fig.3 genommen wird. Wie dieser Fig.4 zu entnehmen ist, lässt sich die Verstärkung für den Sollwert $F_s$ des Gasflusses über einen Block **60** verändern, d.h.der Sollwert kann unterschiedlich verstärkt oder auch gedämpft werden. Aus dem Sollwert $F_s$ und dem Eingangsseitigen Druck $P_a$ wird in einem Block **61**, möglicherweise wieder mit unterschiedlicher Verstärkung, der Sollwert $S_s$ berechnet.

Weiterhin wird, wie bereits beschrieben, der Istwert $F_i$ für den Gasfluss ermittelt. Wie durch einen Pfeil **62** an dem Block **13**, der der Linearisierung dient, angedeutet, kann diese für unterschiedliche Gasgemische variiert werden. Die Blöcke **12** bzw. **15** sind jeweils mit einem Offset versehen. Zusätzlich wird über eine Art Spannungsteiler **19** ein dem Gasdruck $P_a$ entsprechendes Signal dem Block **12** zugeführt und der Differenzdruck $\Delta p$ in Abhängigkeit davon korrigiert (common mode compensation).

In dem Block **63** werden aus dem Soll-und dem Istwert für den Gasfluss der proportionale und der integrale Anteil der Regelgrösse berechnet. Dabei kann die Verstärkung für den intgralen Anteil der Regelgrösse $S_{sI}$ für kleine Gasflüsse erheblich vergrössert werden. Diese werden anschliessend, wie durch den Kreis **64** mit Pluszeichen angedeutet, zusammen mit dem von Block **61** kommenden Signal $S_{sN}$ addiert und als Sollwert $S_s$ auf den Block **65** gegeben, der den zweiten Regelkreis darstellt. Hier wird deutlich, dass dieser Sollwert im wesentlichen aus dem berechneten Positionswert besteht und die über die Flussregelung erhaltenen Regelgrössen zur Feinjustierung dienen. Lediglich bei extrem kleinen Gasflüssen wird, wie aus Fig.2 ersichtlich, der berechnete Positionswert $S_{sN}$ ungenau. Der durch den Block **63** dargestellt erste Regelkreis enthält weiterhin ein Offsetspannung für den Gasfluss, die zum Abgleich herangezogen wird, wenn Soll-und Istfluss Null sind. Auf den Block **65** ist zusätzlich noch der Istwert für die Position $S_i$ geschaltet.Wie durch den Kreis **66** mit Pluszeichen angedeutet, werden der Differnz aus Soll-und Istwerten noch zusätzliche Signale für den Offsetstrom beim Gasfluss Null, dargestellt durch den Block **67**, und über die Leitung **68** der eingangsseitige Gasdruck zur Kompensation der durch diesen ausgeübten Kraft, überlagert.

Die Fig.5 zeigt die impulsbreitenmodulierte Stromsteuerung des Magnetventiles **1**, von dem hier nur die Magnetspule **7** dargestellt ist. Über einen Schalter $S_1$ kann eine Spannung von in diesem Fall +24 V an die Spule angelegt werden. Die an dem in Reihe mit der Spule liegenden Widerstand **71** über einen Verstärker **72** abgegriffene Spannung $U_i$ ist ein Mass für den durch die Spule **7** fliessenden Strom $I_i$. In einem Verstärker **73** wird die Spannungsdifferenz ($U_s$ - $U_i$) verstärkt, wobei $U_s$ dem Sollwert $I_s$ proportional ist, d.h. der Fehler wird verstärkt und als Spannung $U_A$ in dem Komparator **70** mit einer von einem Referenzspannungsgeber **74** erzeugten Sägezahnspannung verglichen. Das Ausgangssignal des Komparators **70** besteht dabei aus Impulsen, deren Impulsdauer mit zunehmendem Fehler grösser wird. Das gilt allerdings nur, solange der Sollwert des Stromes grösser als der Istwert ist. Bei den bisherigen Überlegungen wurde davon ausgegangen, dass der Schalter $S_2$ geschlossen war, wodurch der Strom durch die Spule **7** auch dann aufrecht erhalten bleibt, wenn der Schalter $S_1$ geöffnet wird. Um den Strom durch die Spule konstant zu halten, muss nur die Energie ersetzt werden, die in dem Widerstand und der Diode $D_1$ verbraucht wird. Um den Strom durch die Spule **7** schnell abzusenken, wird der Schalter $S_2$ geöffnet. Der Block **75** ermittelt dazu die erste Ableitung des Sollwertes des Stromes und betätigt bei einer Überschreitung dieser Ableitung über einen vorgegebenen Wert den Schalter $S_2$. Bei offenem Schalter $S_2$ leiten die Dioden $D_1$ und $D_2$ und die magnetische Energie in der Spule **7** wird bis auf die Verluste auf den Kondensator $C_1$ übertragen. Die Spannung über der Spule wird damit etwa -24 V(+ zweimal dem Spannungsabfall über der Diode). Die Energie des Kondensators $C_1$ wird regelmässig, z.B. mit 30 KHz auf den Kondensator $C_2$ überführt, indem synchron die beiden Schalter $S_1$ und $S_2$ kurzzeitig geschlossen werden. Auf diese Weise ist es möglich, den Strom durch die Spule mit geringen Energieverlusten schnell zu erhöhen oder zu senken.

In den abschliessenden Fig.6a-d sind die zeitlichen Verläufe der Soll-und Istwerte für den Strom, die über der Spule **7** liegenden Spannung $U_L$ sowie für die vom Komparator erzeugten Spannungsimpulse $U_k$ dargestellt. Fig.6a zeigt dabei als Beispiel zum Zeitpunkt $t_1$ eine sprunghafte Änderung des Sollwertes für den Strom, die bis zum Zeitpunkt $t_2$ beibehalten wird. Fig. 6b zeigt die vom Komparator **70** gelieferten Spannungsimpulse, die zunächst breit sind und dann immer mehr abnehmen, bis sie schliesslich schmale Spikes sind, mit deren Hilfe nur noch die Verluste in der Spule kompensiert werden.

Mit einer Versorgungsspannung von + 24 V und einer Magnetspule mit einem Widerstand von 2 Ω lassen sich, wie Versuche gezeigt haben, Stromänderungen von etwa 0.5 A/ms erzielen. Das Ventil kann damit von kleinem zu maximalem Gasfluss in etwa 5ms öffnen, eine Regelgeschwindigkeit, die mit keinem der bisher bekannten Systeme möglich ist.

Für den Fall, dass der Strom durch die Spule **7** sehr rasch gesenkt werden muss, ist es mit Hilfe der in Fig. 5 beschriebenen Schaltung einfach möglich, -24V an die Spule anzulegen, was im Beispiel der Fig. 6 zum Zeitpunkt $t_2$ erfolgt.

In den dargestellten Ausführungsbeispielen sind analoge Regelsysteme beschrieben worden. Im Ramen der Erfindung ist es jedoch ebenso möglich, das gesamte Regelsystem oder zumindest Teile davon in Digitaltechnik auszuführen, soweit die dazu notwendigen Komponenten schnell genug arbeiten. Ebenso ist es möglich, die erwähnten vier Parameter zu digitalisieren und die gesamte Regelung über einen Rechner, vorzugsweise einen Mikroprozessor, zu steuern. So ist beispielsweise auch eine Vorprogrammierung des Sollwertes für die Position der Verschlussmittel möglich, so dass diese mit Hilfe des zweiten Regelkreises annähernd in die richtige Position gebracht werden. Die Positionswerte für verschiedene Gasflüsse können dabei beispielsweise in einer Tabelle gespeichert sein.

In allen Fällen erhält man eine Anordnung oder einen Ventilator, die oder der extrem schnell die Durchflussmenge eines fliessenden Mediums mit hoher Genauigkeit und sicher sowohl für grosse als auch kleine Durchflussmengen regelt. Damit ist es beispielsweise bei einem Ventilator möglich, gleichermassen gut kleine Gasflüsse für Kleinkinder als auch grosse Gasflüsse für Erwachsene zu steuern. Der geringe Energieverbrauch erleichtert zusätzlich die mobile Anwendung ohne Anschlussmöglichkeit an herkömmliche Stromnetze oder den Batteriebetrieb z.B. bei Stromausfall. Der Niederspannungsbetrieb erhöht darüber hinaus auch die Sicherheit bei der Anwendung in der Anästhesie, bei der oft explosive Gase verwendet werden. Die hohe Regelgeschwindigkeit ermöglicht eine wesentlich genauere Hochfrequenzventilation.

In den beschriebenen Ausführungsbeispielen steuerte der erste Regelkeis immer den Gasfluss. Im Rahmen der Erfindung ist es aber auch möglich, dass diesem ersten Regelkreis in bekannter Weise mindestens ein anderer Regelkreis vorgeschaltet ist, z.B. einer für der Gasdruck, mit dem ein Patient beatmet werden soll. Dieser bestimmt dann praktisch den Sollwert für den Gasfluss, der notwendig ist, um den gewünschten Gasdruck aufrecht zu erhalten.

**Bezugszeichenliste**

1
Magnetventil
2
Gehäuse
3
Enlass
4
Auslass
5
Ventilöffnung
6
Membran
7
Magnetspule
8
Stange
9
Zugfedern
10

Filter
11
Röhrchenpaket
12
Druckmesser
13
Block zum Linearisieren
14,32
Block zum Nullpunktsabgleich
15
Gasdruckmesser
16-18,21-25,33,41,50-52,68
Leitung
19
Spannungsteiler
20
erster Regelkreis
21,22,23,24,25,33,41
Leitung
30
zweiter Regelkreis
31
Lagesensor
40
weiterer Regelkreis
60
Verstärkerblock
61
Berechnungsblock
62
Pfeil
63
Regelblock
64,66
Kreis
65
Block für zweiten Regelkreis
67
Offset
70
Komparator
71
Widerstand
72,73
Verstärker
74
Referenzspannungsgeber
75
Differenzialwertbildner

**Patentansprüche**

1. Anordnung zur Regelung der Durchflussmenge eines fliessenden Mediums, insbesondere eines Gases, mit einem Ventil (1) mit einem Einlass (3) zur Zufuhr des Mediums und einem Auslass (4) zur Abgabe des Mediums, einer Ventilöffnung (5), bewegbaren Verschlussmitteln zum Verschliessen und Öffnen der Ventilöffnung derart, dass die Grösse der Ventilöffnung (5) veränderbar ist, Mitteln zum Steuern der Lage der Verschlussmittel, einer Vorrichtung zum Bestimmen eines Istwertes der Durchflussmenge, einem Sollwertgeber zum Erzeugen eines Sollwertes für die Durchflussmenge und einem ersten Regelkreis (20) zur Regelung der Durchflussmenge, der aus dem Sollwert und Istwert entsprechend eines negativen Feedbacksystems eine erste Regelgrösse zur Steuerung der Lage der Verschlussmittel derart erzeugt, dass die Differenz zwischen dem Soll- und Istwert gegen Null strebt, **gekennzeichnet durch** einen zweiten Regelkreis (30;40) zur Regelung eines anderen Parameters, wobei die von dem ersten Regelkreis (20) erzeugte Regelgrösse als Sollwert für den zweiten Regelkreis (30;40) verwendet wird.

2. Anordnung zur Regelung der Durchflussmenge eines fliessenden Mediums nach Anspruch 1, **dadurch gekennzeichnet,** dass als Parameter für den zweiten Regelkreis (30;40) die Lage der Verschlussmittel gewählt ist, dass eine Vorrichtung (31) zur Bestimmung des Istwertes für die Lage der Verschlussmittel vorgesehen ist und dass der zweite Regelkreis (30;40) aus der Differenz des Istwertes und der Regelgrösse des ersten Regelkreises (20) als Sollwert für die Lage der Verschlussmittel wiederum entsprechend eines negativen Feedbacksystems eine zweite Regelgrösse erzeugt, die den Mitteln zum Steuern der Lage der Verschlussmittel derart zugeführt wird, dass die Differenz zwischen Soll- und Istwert für die Lage der Verschlussmittel gegen Null strebt.

3. Anordnung nach Anspruch 1 oder 2, **dadurch gekennzeichnet,** dass als Parameter für den zweiten Regelkreis (30;40) der Strom bzw. die Spannung zum Betätigen der Verschlussmittel gewählt ist.

4. Anordnung zur Regelung der Durchflussmenge eines fliessenden Mediums nach Anspruch 1 oder 2 **dadurch gekennzeichnet,** dass als Ventil ein Magnetventil vorgesehen ist, bei dem die Lage der Verschlussmittel durch den Strom oder die Spannung einstellbar ist, mit dem das Ventil (1) beaufschlagt wird, und dass ein weiterer Regelkreis (40;30) vorgesehen ist, dem die zweite Regelgrösse als Sollwert für den Strom oder die Spannung und die über eine Messvorrichtung bestimmte Grösse des Stromes oder der Spannung als Istwert zugeführt werden, und der aus der Differenz zwischen Soll-und Istwert für den Strom oder die Spannung eine weitere Regelgrösse für die Änderung des Stromes oder der Spannung erzeugt derart, dass die Differenz zwischen Soll-und Istwert für den Strom oder die Spannung gegen Null geht.

5. Anordnung nach einem der Ansprüche 1 bis 4, **dadurch gekennzeichnet,** daß das Ventil (1) bei Wegfall einer Regelgrösse automatisch schliesst.

6. Anordnung nach Anspruch 5, **dadurch gekennzeichnet,** dass das Ventil (1) mechanisch mittels Federkraft schliesst.

7. Anordnung nach einem der Ansprüche 3-6, **dadurch gekennzeichnet,** dass das Ventil (1) bei Ausfall des Stromes oder der Spannung zur Einstellung der Lage der Verschlussmittel schliesst.

8. Anordnung nach einem der Ansprüche 3-7, **dadurch gekennzeichnet,** dass der Strom oder die Spannung zur Einstellung der Lage der Verschlussmittel impulsbreitenmodulierbar ist.

9. Anordnung nach einem der Ansprüche 3-8, **dadurch gekennzeichnet,** dass Mittel zur Stromrückgewinnung vorgesehen sind.

10. Anordnung nach einem der Ansprüche 3-9, **dadurch gekennzeichnet,** dass zur schnellen Änderung der Lage der Verschlussmittel, insbesondere bei grösseren Lageänderungen, der Strom oder die Spannung, mit dem oder der das Ventil beaufschlagt wird, umpolbar ist.

11. Anordnung nach Anspruch 10, **dadurch gekennzeichnet,** dass die Verstärkung und/oder die Bandbreite mindestens eines Regelkreises (20,30,40) einstellbar ist.

12. Anordnung nach einem der Ansprüche 1-11, **dadurch gekennzeichnet,** dass die Mittel zum Bestimmen mindestens eines Istwertes analog arbeiten.

13. Anordnung nach einem der Ansprüche 1-12, **dadurch gekennzeichnet,** dass mindestens ein Regelkreis (20;30;40) analog arbeitet.

14. Anordnung nach einem der Ansprüche 1-11, **dadurch gekennzeichnet,** dass mindestens ein Regelkreis (20;30;40) digital arbeitet und der entsprechende Istwert digitalisiert wird.

15. Anordnung nach Anspruch 14, **dadurch gekenn zeichnet,** dass mindestens ein Regelkreis als Mikroprozessor ausgebildet ist.

16. Anordnung nach einem der Ansprüche 1-15, **dadurch gekennzeichnet,** dass der Druck des dem Ventil (1) zugeführten Mediums wesentlich grösser ist als der Druck des vom Ventil (1) abgegebenen Mediums.

17. Verwendung einer Anordnung nach einem der Ansprüche 1-16, in einem Ventilator zum Anschliessen an die Atemwege von Menschen oder Tieren zur ventilgesteuerten Zufuhr und/oder Abgabe von Atemgas bzw. Anästhesigas.

**Claims**

1. Arrangement for controlling the flow rate of a flowing medium, preferably of a gas, having a valve (1) with an inlet (3) for feeding the medium and an outlet (4) for dispensing the medium, a valve opening (5), movable closure means for closing and opening the valve opening such that the size of the valve opening (5) is variable, means for regulating the position of the closure means, a device for determining an actual value of the flow rate, a desired value transmitter for generating a desired value for the flow rate and a first control loop (20) for controlling the flow rate, which generates a first controlled variable for regulating the position of the closure means from the desired value and actual value according to a negative feedback system in such a way that the difference between the desired value and actual value tends to zero, characterized by a second control loop (30; 40) for controlling another parameter, the controlled variable which is generated by the first control loop (20) being used as desired value for the second control loop (30; 40).

2. Arrangement for controlling the flow rate of a flowing medium according to Claim 1, characterized in that the position of the closure means is selected as parameter for the second control loop (30; 40), in that a device (31) for determining the actual value for the position of the closure means is provided, and in that the second control loop (30; 40) generates a second controlled variable from the difference between the actual value and the controlled variable of the first control loop (20) as desired value for the position of the closure means and again according to a negative feedback system, which controlled variable is fed to the means for regulating the position of the closure means in such a way that the difference between desired value and actual value for the position of the closure means tends to zero.

3. Arrangement according to Claim 1 or 2, characterized in that the voltage or the current for actuating the closure means is selected as parameter for the second control loop (30; 40).

4. Arrangement for controlling the flow rate of a flowing medium according to Claim 1 or 2, characterized in that a solenoid valve is provided as valve, in the case of which solenoid valve the position of the closure means can be adjusted by means of the current or the voltage applied to the valve (1), and in that a further control loop (40; 30) is provided to which the second controlled variable is fed as desired value for the current or the voltage, and the value of the current or the voltage as determined via a measuring device is fed as actual value, and which generates, from the difference between desired value and actual value for the current or the voltage, a further controlled variable for the change in the current or the voltage in such a way that the difference between desired value and actual value for the current or the voltage tends to zero.

5. Arrangement according to one of Claims 1 to 4, characterized in that in the case of loss of one controlled variable the valve (1) closes automatically.

6. Arrangement according to Claim 5, characterized in that the valve (1) closes mechanically by means of spring force.

7. Arrangement according to one of Claims 3-6, characterized in that in the case of a failure of the current or the voltage for adjusting the position of the closure means the valve (1) closes.

8. Arrangement according to one of Claims 3-7, characterized in that the current or the voltage for adjusting the position of the closure means is capable of pulse-width modulation.

9. Arrangement according to one of Claims 3-8, characterized in that means for current recovery are provided.

10. Arrangement according to one of Claims 3-9, characterized in that for rapid changes in the position of the closure means, especially for relatively large changes in position, the current or the voltage which is applied to the valve is reversible.

11. Arrangement according to Claim 10, characterized in that the amplification and/or the bandwidth of at least one control loop (20, 30, 40) is adjustable.

12. Arrangement according to one of Claims 1-11, characterized in that the means for determining at least one actual value operates in an analog manner.

13. Arrangement according to one of Claims 1-12, characterized in that at least one control loop (20; 30; 40) operates in an analog manner.

14. Arrangement according to one of Claims 1-11, characterized in that at least one control loop (20; 30; 40) operates in a digital manner and the corresponding actual value is digitized.

15. Arrangement according to Claim 14, characterized in that at least one control loop is designed as a microprocessor.

16. Arrangement according to one of Claims 1-15, characterized in that the pressure of the medium fed to the valve (1) is considerably higher than the pressure of the medium dispensed by the valve (1).

17. Use of an arrangement according to one of Claims 1-16, in a ventilator for connection to the respiratory tract of human beings or animals for valve-regulated feeding and/or dispensing of respiratory gas or anaesthetic gas.

**Revendications**

1. Dispositif pour régler le débit d'un fluide en écoulement, notamment d'un gaz, comportant une vanne (1) ayant une entrée (3) pour l'arrivée du fluide et une sortie (4) pour l'évacuation du fluide, un orifice de vanne (5), des moyens de fermeture mobiles servant à fermer et ouvrir l'orifice de vanne de manière à pouvoir modifier la dimension de l'orifice (5) de la vanne, des moyens destinés à commander la position des moyens de fermeture, un dispositif de détermination de la valeur réelle du débit, un générateur de valeur de consigne destiné à produire une valeur de consigne du débit et un premier circuit de régulation (20) destiné à régler le débit et produisant, à partir de la valeur de consigne et de la valeur réelle, conformément à un système de contre-réaction négative, une première grandeur de régulation destinée à commander la position des moyens de fermeture de sorte que la différence entre la valeur de consigne et la valeur réelle tende à s'annuler, caractérisée par un second circuit de régulation (30; 40) destiné à régler un autre paramètre, la grandeur de régulation produite par le premier circuit de régulation (20) étant utilisée en tant que valeur de consigne pour le second circuit de régulation (30; 40).

2. Dispositif pour régler le débit d'un fluide en écoulement suivant la revendication 1, caractérisé par le fait qu'on choisit, comme paramètre pour le second circuit de régulation (30; 40), la position des moyens de fermeture, qu'il est prévu un dispositif (30) de détermination de la valeur réelle de la position des moyens de fermeture et que le second circuit de régulation (30; 40) produit, à partir de la différence entre la valeur réelle et la grandeur de régulation du premier circuit de régulation (20), à nouveau d'une manière correspondant à un système de contre-réaction négative, en tant que valeur pour la position des moyens de fermeture une seconde grandeur de régulation, qui est envoyée aux moyens destinés à commander la position des moyens de fermeture de telle sorte que la différence entre la valeur de consigne et la valeur réelle de la position des moyens de fermeture tend à s'annuler.

3. Dispositif suivant la revendication 1 ou 2, caractérisé par le fait qu'on choisit comme paramètre pour le second circuit de régulation (30; 40), le courant ou la tension destinée à actionner les moyens de fermeture.

4. Dispositif pour régler le débit d'un fluide en écoulement suivant la revendication 1 ou 2 caractérisé par le fait qu'il est prévu comme vanne une électrovanne, dans laquelle la position des moyens de fermeture est réglable au moyen du courant ou de la tension d'alimentation de la vanne et qu'il est prévu un autre circuit de régulation (40; 30), auquel sont envoyées la seconde grandeur de régulation en tant que valeur de consigne pour le courant ou la tension et la grandeur, déterminée par un dispositif de mesure, du courant ou de la tension en tant que valeur réelle et qui produit, à partir de la différence entre la valeur de consigne et la valeur réelle pour le courant ou la tension, une autre grandeur de régulation pour la variation du courant ou de la tension, de telle sorte que la différence entre la valeur de consigne et la valeur réelle du courant ou de la tension tend à s'annuler.

5. Dispositif suivant l'une des revendications 1 à 4, caractérisé par le fait que la vanne (1) se ferme automatiquement en l'absence d'une grandeur de régulation.

6. Dispositif suivant la revendication 5, caractérisé par le fait que la vanne (1) se ferme mécaniquement sous l'influence d'une force élastique.

7. Dispositif suivant l'une des revendications 3 à 6, caractérisé par le fait que la vanne (1) se ferme dans le cas de l'absence du courant ou de la tension destiné au réglage de la position des moyens de fermeture.

8. Dispositif suivant l'une des revendications 3 à 7, caractérisé par le fait que le courant ou la tension destiné au réglage de la position des moyens de fermeture peut être modulé selon une modulation en largeur d'impulsion.

9. Dispositif suivant l'une des revendications 3 à 8, caractérisé par le fait qu'il est prévu des moyens de récupération du courant.

10. Dispositif suivant l'une des revendications 3 à 9, caractérisé par le fait que pour modifier rapidement la position des moyens de fermeture, notamment dans le cas d'assez grandes variations de position, la polarité du courant ou la tension d'alimentation de la vanne peut être inversée.

11. Dispositif suivant la revendication 10, caractérisé par le fait que l'amplification et/ou la largeur de bande d'au moins un circuit de régulation (20,30, 40) est réglable.

12. Dispositif suivant l'une des revendications 1 à 11, caractérisé par le fait que les moyens de détermination d'au moins une valeur réelle opérent de façon analogique.

13. Dispositif suivant l'une des revendications 1 à 12, caractérisé par le fait qu'au moins un circuit de régulation (20; 30; 40) opére de façon analogique.

14. Dispositif suivant l'une des revendications 1 à 11, caractérisé par le fait qu'au moins un circuit de régulation (20; 30; 40) opère de façon numérique et que la valeur réelle correspondante est numérisée.

15. Dispositif suivant la revendication 14, caractérisé par le fait qu'au moins un circuit de régulation est réalisé sous la forme d'un microprocesseur.

**16.** Dispositif suivant l'une des revendications 1 à 15, caractérisé par le fait que la pression du fluide envoyé à la vanne (1) est nettement supérieure à la pression du fluide fournie par la vanne (1).

**17.** Utilisation d'un dispositif suivant l'une des revendications 1 à 16, dans un respirateur destiné à être raccordé aux voies respiratoires d'hommes ou d'animaux pour l'envoi et/ou l'évacuation, sous la commande d'une vanne, d'un gaz respiratoire ou d'un gaz anesthésique.

# FIG 1

# FIG 2

FIG 3

EP 0 483 401 B1

FIG 4

# FIG 5

# FIG 6